# EUROPÄISCHE PATENTANMELDUNG

(11) **EP 4 310 332 A1**
(43) Veröffentlichungstag der Anmeldung: **24.01.2024**
(21) Anmeldenummer: 23186779.7
(22) Anmeldetag: 20.07.2023
(51) Int. Cl.: F04B 43/12, F04B 43/08, F04B 43/09, A61M 5/142, A61M 60/279, H02N 2/02, H10N 30/20, H10N 39/00

(54) **VERDRÄNGEREINHEIT FÜR EINE MEDIZINISCHE SCHLAUCHPUMPE UND MEDIZINISCHE SCHLAUCHPUMPE**

(30) Priorität: 21.07.2022 DE 102022118250
(71) Anmelder: B. Braun Melsungen AG, 34212 Melsungen (DE)
(72) Erfinder: BARTSCH, Stefan, 36286 Neuenstein (DE)
(74) Vertreter: Winter, Brandl - Partnerschaft mbB

(57) **Zusammenfassung**

Offenbart ist eine Verdrängereinheit für eine medizinische Schlauchpumpe, mit linearbeweglichen Verdrängern, die von einer Antriebseinrichtung der Verdrängereinheit quer zu einer Förderrichtung gegen einen Schlauch betätigbar sind.

Offenbart ist zudem eine medizinische Schlauchpumpe mit einer derartigen Verdrängereinheit.

## Beschreibung

### Technisches Gebiet

Die vorliegende Offenbarung betrifft eine peristaltische Verdrängereinheit für eine medizinische Schlauchpumpe, sowie eine medizinische Schlauchpumpe mit der Verdrängereinheit.

Die Dosierung medizinischer Wirkstoffe oder die Förderung von Blut in der extrakorporalen Blutbehandlung kann über eine Schlauchpumpe erfolgen. Ein Wirkprinzip einer solchen Schlauchpumpe liegt im Allgemeinen darin, einem Schlauch, durch welchen ein Fluid geleitet wird, mittels einer Verdrängereinheit der Schlauchpumpe eine peristaltische Bewegung aufzuprägen, wodurch das Fluid von einem Wirkstoffbehälter hin zu einem Zugang am Patienten gefördert wird.

### Stand der Technik

Eine Schlauchpumpe radialer Bauart ist im Patent EP 3 061 473 B1 der Anmelderin offenbart. Die gezeigte Schlauchpumpe zur extrakorporalen Blutbehandlung hat einen motorischen Drehantrieb mit einem Rotor, an dem radial außen Quetschelemente vorgesehen sind, die bei Rotation wiederkehrend an einem gehäusefest und bogenförmig abgestützten Schlauchabschnitt in einer Schlauchlängsrichtung abgleiten oder abrollen. Auf diese Weise erfährt der Schlauchabschnitt eine periodische, peristaltische Querschnittsverengung, sodass das Blut in Umlaufrichtung des Rotors gefördert wird.

Eine Schlauchpumpe linearer Bauart ist im Patent EP 0 484 717 B1 der Anmelderin gezeigt. Sie hat eine Aufnahme für einen Schlauch, durch welchen Fluid gefördert wird, und eine Verdrängereinheit mit einer Vielzahl schieberartiger Verdränger, die entlang der Aufnahme nebeneinander angeordnet sind und quer zu der Aufnahme linearbeweglich in einem schachtartigen Gehäuseabschnitt geführt sind. Der Aufnahme gegenüberliegende Endabschnitte der Schieber werden von einer rotierenden und am drehbar gelagerten Exzenterwelle betätigt, wobei jedem Schieber ein Exzenter zugeordnet ist und benachbarte Exzenter in Rotationsrichtung der Exzenterwelle einen Winkelversatz aufweisen. Die Rotation der Exzenterwelle bewirkt so die lineare, zeitversetzte Betätigung der Schieber und in Folge die Quetschung des Schlauches nach peristaltischem Muster, was wiederum die Förderung von Fluid zur Folge hat. Der Schacht ist in Betätigungsrichtung der Schieber verschieblich gelagert. Etwaige Toleranzen des Schlauches oder der Schlauchpumpe können durch eine einstellbare Feder ausgeglichen werden, von der die Verdrängereinheit in Betätigungsrichtung der Schieber belastet ist.

Aus dem Sortiment der Anmelderin ist eine Schlauchpumpe unter der Bezeichnung Infusomat ^{®} compact^{plus} bekannt. Auch sie hat eine Verdrängereinheit mit linearbeweglich betätigbaren Schiebern. Endabschnitte der Schieber weisen eine U-förmige Koppelaussparung auf, in die eine von einem Schrittmotor angetriebene Exzenterwelle eingreift. Durch die lineare Führung der Schieber werden die Schieber bei einer Rotation der Exzenterwelle in eine lineare Hin- und Herbewegung versetzt. Eine Stellung der Exzenterwelle wird dabei über eine Lichtschranke mit einer Encoderscheibe überwacht, sodass über eine Steuereinheit der Schlauchpumpe beispielsweise ein Volumenstrom geregelt werden kann. Der Schrittmotor und die davon angetriebene Exzenterwelle sind an einem Gehäuse der Schlauchpumpe schwenkbar derart gelagert, dass die Exzenterwelle über eine einstellbare Feder an die Verdränger angedrückt wird und diese hin zum Schlauch belastet. Somit können auch bei dieser Bauart etwaige Toleranzen des Schlauches oder der Schlauchpumpe durch die einstellbare Feder ausgeglichen werden.

Bei der beschriebenen linearen Bauart ist nachteiliger Weise eine vergleichsweise hohe Anzahl von Bauteilen nötig, um einerseits die peristaltische Bewegung des Schlauches zu bewirken und andererseits den Wirkeingriff der Schieber mit dem Schlauch einzustellen. Mit einer solchen hohen Bauteilanzahl einher gehen hohe Kosten für die Entwicklung und Konzeptionierung der Schlauchpumpe, ein hoher Aufwand zum Einhalten von Toleranzen sowie ein möglicher Verschleiß zwischen den bewegbaren Bauteilen. Zudem beansprucht die Verdrängereinheit aufgrund ihrer hohen Bauteilanzahl nachteiliger Weise einen großen Bauraum.

### Zusammenfassung der Offenbarung

Die Aufgabe der vorliegenden Offenbarung ist es, eine Verdrängereinheit linearer Bauart für eine medizinische Schlauchpumpe zu schaffen, die Nachteile des Stands der Technik reduziert oder ausräumt und die insbesondere gegenüber dem Stand der Technik vorrichtungstechnisch vereinfacht ist. Des Weiteren ist es die Aufgabe der Offenbarung darin, eine entsprechende medizinische Schlauchpumpe linearer Bauart zu schaffen.

Die Aufgabe wird gelöst durch eine Verdrängereinheit für eine medizinische Schlauchpumpe, insbesondere eine als Infusionsgerät oder als Infusionspumpe ausgestaltete medizinische Schlauchpumpe oder eine Schlauchpumpe zur medizinischen Dosierung oder zur extrakorporalen Blutbehandlung, mit linearbeweglich betätigbaren Verdrängern (z.B. Stempel), die mit einer vorbestimmten Abfolge (d.h. zeitversetzt zueinander, insbesondere wellenartig nacheinander) von einer Antriebseinrichtung der Verdrängereinheit zur Erzeugung einer peristaltischen Bewegung eines Schlauches quer zum Schlauch (d.h. quer zu einer Schlauchlängsrichtung, insbesondere senkrecht dazu) und gegen diesen betätigbar sind, um den Schlauch zu quetschen. Die Antriebseinrichtung hat Linearmotoren, die jeweils mit einem der Verdränger gekoppelt sind und von denen zumindest einer (vorzugsweise alle) ein piezoelektrischer Linearmotor ist.

In anderen Worten ausgedrückt, ist eine Verdrängereinheit, auch Peristaltikeinheit genannt, die für eine medizinische Schlauchpumpe, beispielsweise eine Dosierpumpe oder eine Dialysepumpe, vorgesehen. Die Verdrängereinheit hat eine Vielzahl (zumindest zwei, vorzugsweise vier bis sechszehn, weiter vorzugsweise sechs bis zwölf) linearbeweglich betätigbarer Verdränger, die in Anlage mit dem fluid- oder blutführenden Schlauch der Schlauchpumpe sind oder bringbar sind und an diesen anpressbar sind, wodurch dem Schlauch eine peristaltische Bewegung im Sinne einer fortschreitenden Querschnittsverengung aufgeprägt wird. Die Verdränger sind zu diesem Zweck von einer Antriebseinrichtung der Verdrängereinheit in einer, insbesondere vorbestimmten, Abfolge quer gegen den Schlauch betätigbar. Offenbarungsgemäß weist die Antriebseinrichtung piezoelektrische Linearmotoren auf, die jeweils mit einem der linearbeweglichen Verdränger gekoppelt sind. Der Begriff des Linearmotors ist in dieser Schrift so definiert, dass die von ihm bewirkte Linearbewegung der Verdränger ohne eine drehende Welle erzeugt ist. Die Verdränger sind insbesondere passive Bauteile oder Stempel, welche mechanisch, insbesondere rein mechanisch, mit den jeweiligen Antriebseinrichtungen gekoppelt sind.

Auf diese Weise ist eine vorrichtungstechnisch vereinfachte Verdrängereinheit geschaffen, da die Verdränger vom Linearmotor direkt in ihrer linearen Betätigungsrichtung angetrieben sind und die Notwendigkeit entfällt, eine rotatorische Antriebsbewegung in die gewünschte Linearbewegung der Verdränger zu wandeln. Dadurch können die für eine solche Wandlung vorzusehenden Bauteile entfallen. Gegenüber den einleitend geschilderten Beispielen gemäß dem Stand der Technik bedeutet das konkret, dass ein Schrittmotor, sowie eine Exzenterwelle oder ein anderes entsprechendes Getriebe zur Wandlung der Rotationsbewegung in die Linearbewegung überflüssig sind. Bereits diese Reduzierung der Bauteilanzahl reduziert den von der Verdrängereinheit benötigten Bauraum erheblich. In Folge entfallen zudem eine Lagerung der Exzenterwelle/des Getriebes, sowie Koppelstellen oder Reibverbindungen der Exzenterwelle/des Getriebes mit den Verdrängern, wodurch ein Verschleiß reduziert ist. Aufgrund der Reduzierung der Bauteilanzahl ist zudem ein Aufwand zur Einhaltung von Toleranzen reduziert. Zudem bauen piezoelektrisch Linearmotoren besonders kompakt, weisen einen sehr geringen Verschleiß auf und können sehr gut in Hub, Hubgeschwindigkeit und Hubkraft gesteuert und/oder geregelt werden.

In einer bevorzugten Weiterbildung ist der jeweilige Verdränger mit mehr als nur einem piezoelektrischen Linearmotor gekoppelt. Diese Linearmotoren können an einer Seite des Verdrängers parallel oder in Reihe zueinander (in Bezug auf eine Bewegungsrichtung des Verdrängers) und/oder auf mehrere Seiten des Verdrängers (insbesondere auf einander gegenüberliegenden Seiten) verteilt angeordnet sein. So kann eine Betätigungskraft erhöht werden und eine Kraftübertragung auf den Verdränger ausbalanciert werden. Dies mindert eine Gefahr eines Verkantens des Verdrängers, was die Möglichkeit birgt, Aufwände zu dessen Lagerung zu verringern.

In einer bevorzugten Weiterbildung ist ein jeweiliger Hub, eine jeweilige Hubgeschwindigkeit und/ oder eine jeweilige Kraft der Linearmotoren, insbesondere ein jeweiliger zeitlicher Verlauf des Hubes oder der Kraft, steuerbar oder regelbar. Verglichen mit den Beispielen des eingangs geschilderten Standes der Technik kann so eine aufwändige Mechanik zum Einstellen der auf den Schlauch aufgebrachten Vorspannung der Verdränger entfallen. Die Einstellung kann somit anstatt herkömmlich durch die mechanisch bewirkte Vorspannung durch die direkte Ansteuerung der Linearmotoren erfolgen. Dies bringt eine erhebliche Einsparung an Bauteilen und Bauraum mit sich, wodurch die Verdrängereinheit, und damit natürlich auch die sie beinhaltende Schlauchpumpe, noch kleiner bauen kann.

Bevorzugt sind die Linearmotoren in Bezug auf eine Bewegungsrichtung der Verdränger jeweils seitlich von den Verdrängern angeordnet. Dadurch kann besonders in der Bewegungsrichtung der Verdränger ein Bauraum erheblich reduziert werden.

Besonders bevorzugt haben die Verdränger jeweils zumindest eine Seitenkante, welche zum Reib- und/oder Formschluss mit zumindest einem Aktorelement, insbesondere einem amorphen Biegeaktor, der Linearmotoren ausgebildet sind, wobei die Seitenkante insbesondere flach ist. Dies ermöglicht es, die Verdränger und die zugehörigen Linearmotoren in Schlauchlängsrichtung besonders nahe aneinander anzuordnen, sodass ein Bauraum weiter reduziert ist und eine besonders gleichmäßige peristaltische Bewegung im Schlauch erzeugt werden kann. Bevorzugt sind die Verdränger scheibenartige Stempel deren flache Seiten einander zugewandt sind.

In einer bevorzugten Weiterbildung weist die Verdrängereinheit eine Tragstruktur oder eine Gehäusestruktur auf, in oder an der die Verdränger linearbeweglich und/ oder Statoren der Linearmotoren fest gelagert sind. Die Trag- oder Gehäusestruktur ermöglicht eine modulartige und daher einfache Montage der Verdrängereinheit in der Schlauchpumpe. Bevorzugt ist zumindest ein seitlicher Rand der Verdränger, insbesondere angrenzend an die Seitenkante, als zumindest eine Führungsschiene ausgebildet, welche in/ an der Tragstruktur längsbeweglich geführt ist. Somit muss nur ein (oder ggf. zwei) Rand und die zugehörige Kante nachbearbeitet werden und können Fertigungskosten reduziert werden.

In einer bevorzugten Weiterbildung hat die Gehäuse- oder Tragstruktur Verbindungsschnittstellen, die so ausgestaltet sind, dass über sie die Verdrängereinheit mit der medizinischen Schlauchpumpe in allen Freiheitsgraden bestimmt verbindbar ist.

Vorzugsweise hat die Tragstruktur oder die Gehäusestruktur einen oder mehrere Schächte, in denen die Verdränger linearbeweglich gelagert sind. Deren Lagerung kann beispielsweise über ein jeweiliges Gleitlager oder eine Gleitlageranordnung erfolgen.

Zur Höhenführung oder -lagerung der Verdränger im Schacht weist dieser in einer Weiterbildung wenigstens einen Führungsrand oder eine Führungsfläche auf, an dem/ der die Verdränger linearbeweglich gelagert sind. Vorzugsweise sind zwei einander gegenüberliegende Führungsränder oder -flächen vorgesehen, zwischen denen die Verdränger angeordnet und an denen sie linearbeweglich gelagert sind.

In einer Weiterbildung weist der Schacht Führungslamellen oder -stege auf, die sich ausgehend von dem/ der wenigstens einen Führungsrand/ -fläche, vorzugsweise ausgehend von beiden, in den Schacht hinein erstrecken. Über die Führungslamellen oder -stege sind die Verdränger (z.B. an einer oder beiden Seiten der Führungsschiene) im Schacht lateral geführt und auf Abstand gehalten, sodass ihre unabhängige, störungsfreie Betätigung sichergestellt ist.

Eine medizinische Schlauchpumpe, insbesondere eine als Infusionsgerät oder als Infusionspumpe ausgestaltete medizinische Schlauchpumpe oder eine Schlauchpumpe zur medizinischen Dosierung oder zur extrakorporalen Blutbehandlung, hat ein Gehäuse mit einer außenseitig zugänglichen Durchgangsausnehmung, die von einem fluid- oder blutführenden Schlauch überspannbar oder in die der Schlauch einlegbar ist. Vorzugsweise sind am Gehäuse seitlich der Durchgangsausnehmung diametral angeordnete, außenseitig zugängliche Aufnahmen ausgebildet, in die je ein Koppelabschnitte eines fluid- oder blutführenden Schlauches aufnehmbar und insbesondere formschlüssig haltbar ist. Im Gehäuse ist eine peristaltische Verdrängereinheit aufgenommen, die gemäß wenigstens einem Aspekt der vorangegangenen Beschreibung ausgestaltet ist und deren Verdränger zur Erzeugung der peristaltischen Bewegung des Schlauches durch die Durchgangsausnehmung hindurch oder im Bereich der Durchgangsausnehmung in Wirkeingriff mit dem Schlauch bringbar sind.

Wie bereits weiter oben beschrieben, sind die Verdränger der in der Schlauchpumpe verbauten Verdrängereinheit linearmotorisch betätigt, wodurch sich die bereits genannten Vorteile der Verdrängereinheit ergeben, auf die an dieser Stelle verwiesen wird. In Folge dessen ist auch die Schlauchpumpe vorrichtungstechnisch vereinfacht aufgebaut, beansprucht weniger Bauraum, weist weniger Verschleiß auf und der Aufwand zur Wahrung interner Toleranzen ist verringert.

Um einen medizinischen Behandlungs- oder Einsatzraum vom maschinellen Gehäuseinnenraum der Schlauchpumpe sicher zu trennen, ist in einer Weiterbildung die Durchgangsausnehmung von einer Membran überspannt, gegen die innenseitig die Verdränger wirksam sind.

### Figurenbeschreibung

Die Offenbarung wird im Folgenden anhand einer beispielhaften, nicht einschränkenden und in den Figuren gezeigten Ausführungsform näher erläutert. Dabei zeigt:
Figur 1 eine medizinische Schlauchpumpe gemäß einem Ausführungsbeispiel in einer Ansicht von vorne,
Figur 2 die Schlauchpumpe gemäß Figur 1 in einer perspektivischen Ansicht von vorne,
Figur 3 eine peristaltische Verdrängereinheit der Schlauchpumpe gemäß Figur 1 und 2 in einer perspektivischen Ansicht,
Figur 4 die Verdrängereinheit gemäß Figur 3 in einer Ansicht von vorne,
Figur 5 Verdränger der Verdrängereinheit gemäß den Figuren 3 und 4 freigestellt in einer Ansicht von vorne,
Figur 6 die Verdränger gemäß Figur 5 in einer Ansicht von oben,
Figur 7 die Verdrängereinheit gemäß Figur 3, in einer perspektivischen Ansicht von hinten, und
Figur 8 eine Kontaktzone eines der Verdränger mit einem ihm zugeordneten Linearmotor gemäß den Figuren 3 bis 7, in einer perspektivischen Detailansicht.

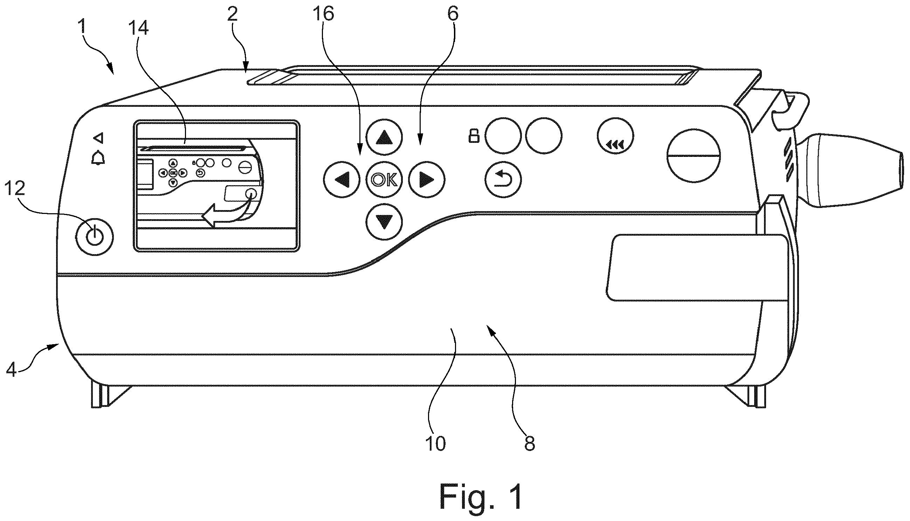

Figur 1 zeigt eine als Infusionsgerät ausgestaltete medizinische Schlauchpumpe 1 in einer Ansicht von vorne. Das Infusionsgerät bzw. die Schlauchpumpe 1 hat ein im wesentlichen quaderförmiges Gehäuse 2 mit einer Frontseite 4, die im Wesentlichen in ein oberes Bedienfeld 6 und ein unteres, verschließbares Schlauchfach 8 unterteilt ist. Letztgenanntes ist von einem klappbaren Schlauchfachdeckel 10 überdeckt.

Das Bedienfeld 6 hat eine Vielzahl Taster zur Bedienung des Infusionsgerätes 1 und umfasst unter anderem einen An-/Aus-Taster 12 ein Display 14 zur Darstellung von gewählten Betriebsparametern, aktuellen Betriebsgrößen und eines Bedienungsmenüs, eine Tasteranordnung 16 zur Navigation im Bedienungs-Menü und zur Änderung von Betriebsparametern, sowie weitere Taster zur Bedienung.

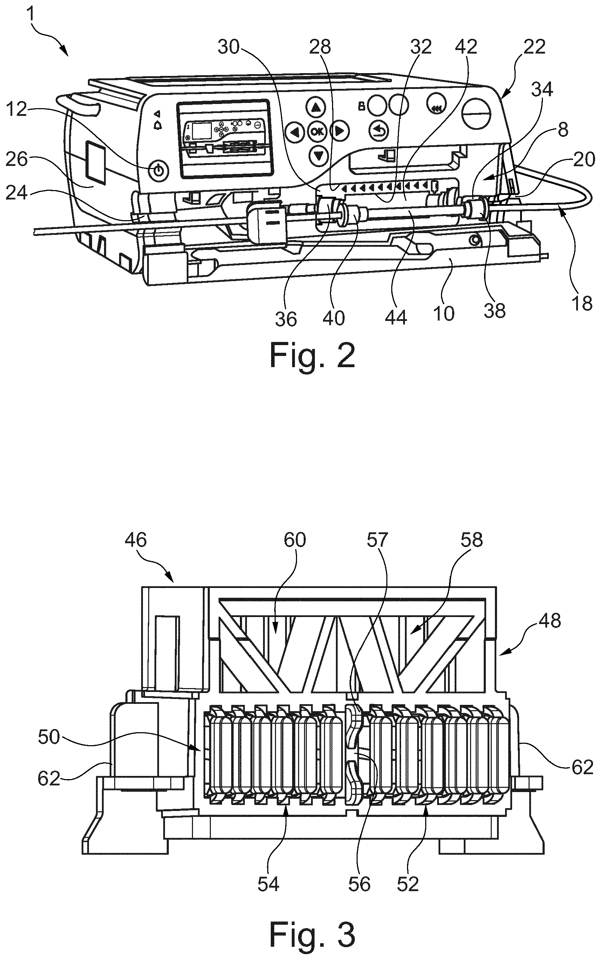

Figur 2 zeigt das Infusionsgerät 1 gemäß Figur 1 in einer perspektivischen Ansicht von vorne mit geöffnetem Schlauchfach 8, also mit aufgeklapptem Schlauchfachdeckel 10. Dabei erstreckt sich gemäß Figur 2 von rechts, ausgehend von einem Infusionsbehälter (nicht dargestellt), ein Schlauch oder Infusionsschlauch 18 in das Schlauchfach 8 hinein. Der Infusionsschlauch 18 ist dabei noch nicht bestimmungsgemäß eingelegt, sondern ist in einer - mit Bezug zur Betriebslage - abgehobenen Lage dargestellt.

Das Schlauchfach 8 hat einen Schlaucheingang 20 im Bereich einer aus Bedienungssicht rechten Seitenwand 22, sowie einen Schlauchausgang 24 im Bereich einer linken Seitenwand 26.

Der gemäß Figur 2 niedergeklappte Schlauchfachdeckel 10 bildet zusammen mit einem Boden oder Grund des Schlauchfaches 8 Formabschnitte, also unterschiedlich geformte und zur Anlage an den Infusionsschlauch angepasste Abschnitte, aus, in die der Infusionsschlauch 18 gehäusefest in seine Betriebslage einlegbar ist. Durch das Schließen des Schlauchfachdeckels 10 wird der Infusionsschlauch 18 in dieser Betriebslage fixiert.

Mit Bezug zur Förderrichtung von rechts nach links gemäß den Figuren 1 und 2, ist rechts am Grund des Schlauchfaches 8 eine im Wesentlichen rechteckige Aussparung 28 im Gehäuse 2 vorgesehen, in der ein Adapterabschnitt 30 des Gehäuses 2 angeordnet ist. Diese Aussparung 28 hat eine Durchgangsausnehmung 32, rückwärtig von der Verdränger 50 einer peristaltischen Verdrängereinheit 46 angeordnet sind, welche in den nachfolgenden Figuren näher erläutert wird.

Beidseitig der Durchgangsausnehmung 32 in einer Erstreckungsrichtung des Infusionsschlauches 18, wenn dieser in die Schlauchpumpe 1 eingelegt ist (d.h. in einer Schlauchlängsrichtung), ist jeweils eine sich zur Durchgangsausnehmung 32 hin stufig verjüngende, halbschalenförmige oder halbzylindrische Aufnahme 34, 36 für einen Koppelabschnitt 38, 40 des Infusionsschlauches 18 vorgesehen. Die Durchgangsausnehmung 32 ist gehäuseinnenseitig von einer Membran 42 überspannt, die einen Gehäuseinnenraum des Infusionsgerätes 1 vom medizinischen Behandlungsraum separiert, beziehungsweise umgekehrt, den Gehäuseinnenraum gegen von außen eindringendes Fluid, Desinfektionsmittel oder Verschmutzung schützt.

Gemäß Figur 2 sind die Koppelabschnitte 38, 40 des Infusionsschlauchs 18 in die Aufnahmen 34, 36 einsetzbar, sodass ein zwischen den Koppelabschnitten 38, 40 ausgebildeter Schlauch- oder Peristaltikabschnitt 44 des Infusionsschlauchs 18 linear/ gerade verlaufend in der Durchgangsausnehmung 32 gehalten ist. Der Schlauchfachdeckel 10 ist durch Hochklappen schließbar. Dadurch ist der Infusionsschlauch 18 formschlüssig im Schlauchfach 8 aufgenommen und vom als Widerlager wirkenden Schlauchfachdeckel 10 eingespannt. Hinter der Membran 42 angeordnete Verdränger 50 der Schlauchpumpe (vgl. folgende Figuren) können dann dem Infusionsschlauch 18, genauer gesagt dem sich zwischen den Koppelabschnitten 38, 40 erstreckenden Peristaltikabschnitt 44, eine peristaltische Verengung seines Querschnittes aufprägen. Alternativ zum dargestellten, mehrstückig ausgebildeten Schlauch/Infusionsschlauch 18 kann dieser einstückig sein. Im ersten Fall kann mit einem gezielt ausgewählten Material des von den Verdrängern zu belasteten Schlauchabschnitts, beispielsweise Silikon, auf mechanische Anforderungen reagiert werden. Die Verwendung eines einstückigen Schlauches ist hingegen vorrichtungstechnisch einfacher.

Figur 3 zeigt die bereits in der Beschreibung zur Figur 2 erwähnte Verdrängereinheit 46 des Infusionsgerätes 1. Die Verdrängereinheit 46 hat eine Tragstruktur 48 und darin parallel zueinander linearbeweglich aufgenommene Verdränger 50. Diese sind flach ausgebildet und weisen, der Membran 42 gemäß Figur 2 zugewandte, konisch zulaufende und verrundete Betätigungskanten auf, die in Anlage mit der Membran 42 und somit in Wirkeingriff mit dem Infusionsschlauch 18 gemäß Figur 2 bringbar sind. Dabei ist der von den Betätigungskanten eingenommene Querschnitt etwas kleiner als derjenige der Durchgangsausnehmung 32 gemäß Figur 2.

Die Tragstruktur 48 hat einen im wesentlichen quaderförmigen Aufbau. In Erstreckungsrichtung des Infusionsschlauches, also in Figur 3 von rechts nach links, sind nebeneinander zwei Schächte 52 und 54 vorgesehen, in denen jeweils sechs Verdränger 50 angeordnet sind. Die Schächte 52, 54 sind durch eine Trennwand 56 getrennt, aus der ein Stützelement 57 auskragt, welches bei einer vergleichsweise weichen Bauform des Infusionsschlauches 18 diesem eine Höhenführung gibt. Oberhalb der beiden Schächte 52, 54 ist ein Aktuatorraum 58 in der Tragstruktur 48 ausgebildet, in dem piezoelektrische Linearmotoren 60 gehäusefest aufgenommen sind. Jeder der Verdränger 50 ist dabei mit einem der Linearmotoren 60 gekoppelt.

Die piezoelektrischen Linearmotoren 60 sind im gezeigten Ausführungsbeispiel als Schreitmotoren ausgebildet. Davon abweichende Bauformen sind möglich.

Seitlich an der Tragstruktur 48 kragen Befestigungsabschnitte 62 aus, über die die Verdrängereinheit 46 im Gehäuse 2 des Infusionsgerätes 1 befestigbar ist.

Gut zu erkennen ist in Figur 3 eine vergleichsweise geringe Bautiefe der Verdrängereinheit 46. Diese wird ermöglicht, indem die Verdränger 50 offenbarungsgemäß linearmotorisch - und somit in ihrer gewünschten Betätigungsrichtung direkt - angetrieben sind, wodurch eine Getriebevorrichtung zur Wandlung einer rotatorischen in die lineare Bewegung der Verdränger 50 entfällt.

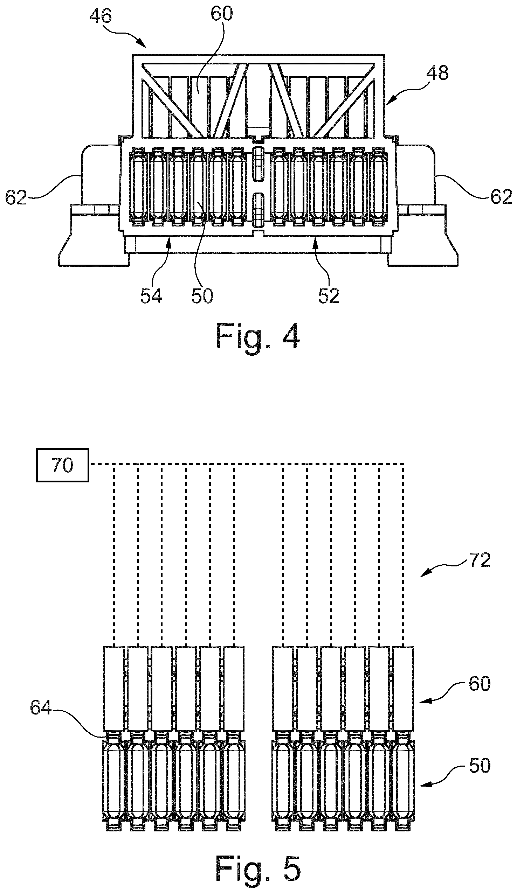

Figur 4 zeigt die Verdrängereinheit 46 gemäß Figur 3 in einer Ansicht von vorne, das heißt in einer Ansicht, wie sich von vorne auf das Schlauchfach 8 gemäß Figur 2 bei entferntem Gehäuse 2 ergibt. Dabei ist gut zu erkennen, dass die Schächte 52, 54 je eine obere und eine untere Führungsfläche aufweisen, zwischen denen die Verdränger 50 gleitend gelagert angeordnet sind. Aus den Führungsflächen heraus erstrecken sich kurze Führungslamellen oder -stege, an bzw. zwischen denen die Verdränger 50 lateral gleitend geführt sind.

Figur 5 zeigt die von der Tragstruktur 48 freigestellten Einheiten aus Verdrängern 50 und Linearmotoren 60 gemäß den Figuren 3 und 4, wodurch eine Kontaktzone 64 des jeweiligen Linearmotors 60 mit dem ihm zugeordneten Verdränger 50 sichtbar ist.

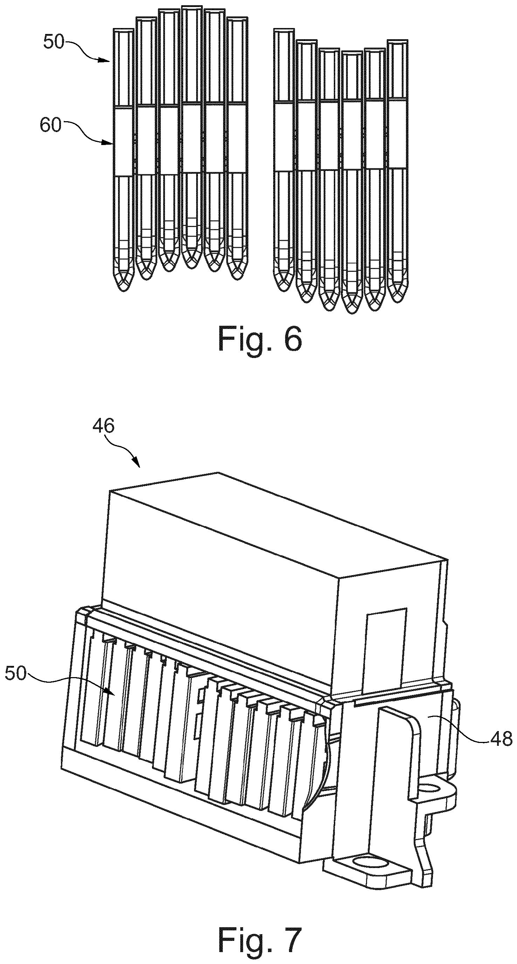

Zur Erzeugung der notwendigen peristaltischen Bewegung des Infusionsschlauches 18 wird den Verdrängern 50 über die ihnen zugeordneten Linearmotoren 60 in einer vorbestimmten Abfolge ein etwa sinusförmiges, peristaltisches Bewegungsmuster alternierend aufgeprägt, was in Figur 6 zu einem Zeitpunkt t dargestellt ist.

Die Schlauchpumpe 1 hat eine Steuereinheit 70, von der die Linearmotoren 60 über Signalleitungen 72, insbesondere in Abhängigkeit der über das Bedienfeld 6 vorgegebenen Parameter, ansteuerbar sind. In der Steuereinheit sind als Parameter eine Abfolge der Betätigung der Verdränger 50, deren Hub, Hubgeschwindigkeit und/oder Hubkraft abgelegt. Diese Parameter sind über die Taster des Bedienfeldes 6 änderbar.

Figur 7 zeigt die Verdrängereinheit 46 in einer Ansicht perspektivisch von hinten, was erneut ihre vergleichsweise geringe Bautiefe und den kleinen, von ihr beanspruchten Bauraum verdeutlicht.

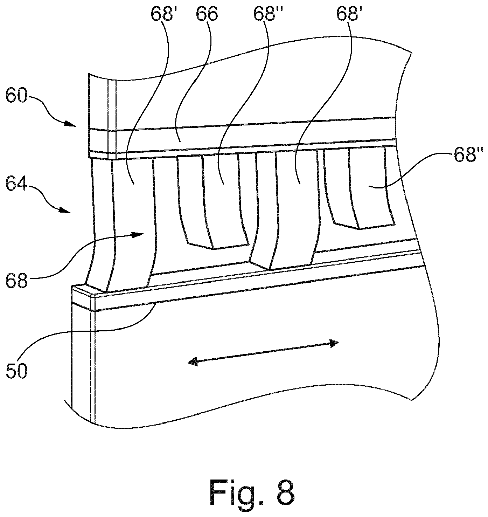

Figur 8 zeigt in schematisierter Darstellung eine Detailansicht der Kontaktzone 64 zwischen einem der Verdränger 50 und dem mit ihm gekoppelten piezoelektrischen Linearmotor 60. Dieser ist als Schreitmotor ausgeführt und hat einen Stator mit einem Statorgehäuse 66. Für seine Ansteuerung und Versorgung mit elektrischer Antriebsenergie ist der Linearmotor über eine Kontaktfahne mit der Steuereinheit der Schlauchpumpe elektrisch verbunden (nicht dargestellt). In der Ausgestaltung als piezoelektrischer Schreitmotor kragen aus dem Statorgehäuse 66 eine Vielzahl von bimorphen Biegeaktoren 68, die in und entgegen der Betätigungsrichtung der Verdränger 50 (entlang des Doppelpfeils) verformbar und quer dazu abhebbar sind. Im vorliegenden Beispiel sind die Biegeaktoren 68 derart verformbar, dass ihre freien Enden, welche zum Reibkontakt mit dem Verdränger 50 ausgebildet sind, abhängig von einer Bestromung der Biegeaktoren 68 um einen bestimmten Winkel klappbar sind.

Ergänzend zu den in Figur 8 gezeigten, an nur eine Oberseite der Verdränger 50 angeordneten Linearmotoren 60 können diese natürlich auch an einer zusätzlichen Seite der Verdränger 50, beispielsweise deren Unterseite angeordnet sein. Die Anordnung kann dabei spiegelbildlich ausgebildet sein.

Zum in Figur 8 dargestellten Zeitpunkt sei die Betätigungsrichtung hin zum Infusionsschlauch von links nach rechts angenommen. Durch entsprechende Bestromung sind zwei Biegeaktoren 68" abgehoben und zwei andere Biegeaktoren 68` mit dem ihnen maximal möglichen Rückhub (d.h. einer maximal entgegen der Betätigungsrichtung zurückgesetzten Stellung), entgegen der Betätigungsrichtung, verformt und auf dem Verdränger 50 aufgesetzt.

Bei einer Änderung der Ansteuerung bleiben die beiden letztgenannten Biegeaktoren 68` auf den Verdränger 50 aufgesetzt und werden in Figur 8 derart nach rechts verformt, d.h. in einen maximal möglichen Vorhub gebracht. Dies bewirkt über die auf den Verdränger 50 übertragene Reibkraft eine Linearbewegung des Verdrängers 50 in Figur 8 nach rechts, hin zum Infusionsschlauch 18.

An ihrem in Betätigungsrichtung maximal möglichen Hub (Vorhub) angekommen, bleiben die beiden Biegeaktoren 68` aufgesetzt und die beiden anderen 68" werden in abgehobener Stellung in ihren maximal möglichen Rückhub, entgegen der Betätigungsrichtung, verformt und dann aufgesetzt.

In diesem Zustand sind kurzzeitig alle Biegeaktoren 68` und 68" aufgesetzt, die erstgenannten Biegeaktoren 68` mit maximal möglichem Hub in Betätigungsrichtung, die anderen Biegeaktoren 68" mit maximal möglichem Hub entgegen der Betätigungsrichtung. So wird der Verdränger 50 von den einen Biegeaktoren 68` an die anderen Biegeaktoren 68" "übergeben".

Es erfolgt das Abheben der einen Biegeaktoren 68`, deren Verformung entgegen der Betätigungsrichtung von rechts nach links, sowie das Verformen der anderen aufgesetzten Biegeaktoren 68" in Betätigungsrichtung von links nach rechts.

Die beschriebene Ansteuerung erfolgt für alle Linearmotoren 60 in einer in der Steuereinheit zur Ausführung gespeicherten Abfolge in Abhängigkeit wenigstens eines der Parameter Hub, Hubgeschwindigkeit und Hubkraft. Die Steuereinheit ist insbesondere dafür ausgelegt, die Parameter aus einem vom Bediener/von der Bedienerin vorgegebenen Volumenstrom zu ermitteln.

Durch den beschriebenen Einsatz der piezoelektrischen Linearmotoren 60 kann das Prinzip der nebeneinander angeordneten Verdränger 50 realisiert werden, die eine Peristaltikbewegung ausführen bzw. auf den Infusionsschlauch 18 aufprägen. Die jeweiligen Biegeaktoren 68 des jeweiligen piezoelektrischen Linearmotors 60 "laufen" dabei auf dem zugeordneten Verdränger 50 und bewegen diesen in der Betätigungsrichtung hin oder zurück, je nach zeitlicher Ansteuerung über die Steuereinheit.

Ein bedeutender Vorteil der beschriebenen Bauform der Verdrängereinheit 46 besteht in ihrer Zuverlässigkeit und Lebensdauer. Die auf kristallinen Strukturen basierende Bauform des piezoelektrischen Linearmotors 60 unterliegt nur wenig oder keiner wesentlichen Abnutzung. Ein Platzbedarf des Linearmotors 60, insbesondere seine Bauhöhe und/ oder Baubreite ist gering, und seine Steuerbarkeit ist hochauflösend und kann beispielsweise im Nanometerbereich liegen. Auf diese Weise kann die Position des Verdrängers 50 relativ zum (Infusions-) Schlauch 18 im Nanometerbereich gesteuert werden. In Folge kann auch die Kraft, die der Verdränger 50 auf den Schlauch 18 ausübt, und in Folge der Druck im Schlauch 18 äußerst exakt beeinflusst werden. Auch die erreichbaren Geschwindigkeiten sind hoch. So sind beispielsweise Linearbewegungen mit 500mm/sec möglich. Prinzipiell ist ein Vorteil dieser Bauform ein geringer Energiebedarf durch weniger Komponenten und Reibung. Da für die gleiche Funktion wie im Stand der Technik weniger Bauteile benötigt werden, ergibt sich auch ein geringerer benötigter Bauraum, sowie ein geringeres Gewicht, sowohl der Verdrängereinheit 46, als auch der Schlauchpumpe 1, in der die Verdrängereinheit 46 eingesetzt ist. Ein weiterer Vorteil ist ein geringerer Montageaufwand, der aus der verringerten Bauteilanzahl resultiert.

Offenbart ist zusammenfassend eine peristaltisch arbeitende Verdrängereinheit für eine medizinische Schlauchpumpe mit mehreren Linearmotoren zur Erzeugung einer Peristaltikbewegung eines fluidführenden Schlauches, sodass dieser in eine bestimmungsgemäße Richtung geknetet wird und so das Fluid fördert.

### Referenzzeichenliste

- 1: Schlauchpumpe
- 2: Gehäuse
- 4: Frontseite
- 6: Bedienfeld
- 8: Schlauchfach
- 10: Schlauchfachdeckel
- 12: Taster
- 14: Display
- 16: Tasteranordnung
- 18: Schlauch
- 20: Schlaucheingang
- 22: Seitenwand
- 24: Schlauchausgang
- 26: Seitenwand
- 28: Aussparung
- 30: Adapterabschnitt
- 32: Durchgangsausnehmung
- 34, 36: Aufnahme
- 38, 40: Koppelabschnitt
- 42: Membran
- 44: Schlauchabschnitt
- 46: Verdrängereinheit
- 48: Tragstruktur
- 50: Verdränger
- 52, 54: Schacht
- 56: Trennwand
- 57: Stützelement
- 58: Aktuatorraum
- 60: piezoelektrischer Linearmotor
- 62: Befestigungsabschnitt
- 64: Kontaktzone
- 66: Statorgehäuse
- 68: Biegeaktor
- 70: Steuereinheit
- 72: Signalleitungen

## Patentansprüche

1. Verdrängereinheit (46) für eine medizinische Schlauchpumpe (1), insbesondere eine als Infusionsgerät oder als Infusionspumpe ausgestaltete medizinische Schlauchpumpe (1) oder eine Schlauchpumpe (1) zur medizinischen Dosierung oder zur extrakorporalen Blutbehandlung, mit linearbeweglich betätigbaren Verdrängern (50), die mit einer vorbestimmten Abfolge von einer Antriebseinrichtung (60) der Verdrängereinheit (46) zur Erzeugung einer peristaltischen Bewegung eines Schlauches oder Schlauchabschnitts (44) quer zum Schlauch oder Schlauchabschnitt (44) und gegen diesen betätigbar sind, um den Schlauch oder Schlauchabschnitt (44) zu quetschen, **dadurch gekennzeichnet, dass** die Antriebseinrichtung Linearmotoren (60) hat, die jeweils mit einem der Verdränger (50) gekoppelt sind und von denen zumindest einer ein piezoelektrischer Linearmotor (60) ist.

2. Verdrängereinheit (46) nach Anspruch 1, wobei wenigstens einer der Linearmotoren ein piezoelektrischer Schreitmotor (60) ist.

3. Verdrängereinheit (46) nach Anspruch 1 oder 2, wobei die Linearmotoren (60) parallel zueinander angeordnet und beweglich sind.

4. Verdrängereinheit (46) nach einem der vorstehenden Ansprüche 1 bis 3, wobei die Linearmotoren (60) in Bezug auf eine Bewegungsrichtung der Verdränger (50) jeweils seitlich von den Verdrängern (50) angeordnet sind.

5. Verdrängereinheit (46) nach einem der vorstehenden Ansprüche 1 bis 4, wobei die Verdränger (50) jeweils eine Seitenkante aufweisen, welche zum Reib- und/oder Formschluss mit zumindest einem Aktorelement, insbesondere einem amorphen Biegeaktor (68), der Linearmotoren (60) ausgebildet sind, wobei die Seitenkante insbesondere flach ist.

6. Verdrängereinheit (46) nach einem der Ansprüche 1 bis 5, ferner mit einer Tragstruktur (48), in oder an der die Verdränger (50) linearbeweglich gelagert sind, wobei zumindest ein seitlicher Rand der Verdränger (50), insbesondere angrenzend an die Seitenkante, als zumindest eine Führungsschiene ausgebildet ist, welche in oder an der Tragstruktur (48) längsbeweglich geführt ist.

7. Verdrängereinheit (46) nach einem der Ansprüche 1 bis 5, wobei die Tragstruktur (48) zumindest einen Schacht (52, 54) ausbildet, in dem die Verdränger (50) linearbeweglich gelagert und zueinander beabstandet sind.

8. Verdrängereinheit (46) nach einem der vorhergehenden Ansprüche mit einer Steuereinheit (70), über die ein Hub oder eine Kraft wenigstens eines der Verdränger (50) steuerbar oder regelbar ist.

9. Verdrängereinheit nach einem der vorhergehenden Ansprüche mit einer Gehäuse- oder Tragstruktur (48), in oder an der die Verdränger (50) linearbeweglich gelagert sind, und an der Statoren (66) der Linearmotoren (60) befestigt sind.

10. Medizinische Schlauchpumpe (1), insbesondere eine als Infusionsgerät oder als Infusionspumpe ausgestaltete medizinische Schlauchpumpe (1) oder eine Schlauchpumpe (1) zur medizinischen Dosierung oder zur extrakorporalen Blutbehandlung, mit einem Gehäuse (2) mit einer außenseitig zugänglichen Durchgangsausnehmung (32), in oder über die ein fluid- oder blutführenden Schlauch (18) oder Schlauchabschnitt (44) einlegbar oder spannbar ist, wobei im Gehäuse (2) eine gemäß einem der vorhergehenden Ansprüche ausgebildete Verdrängereinheit (46) aufgenommen ist, deren Verdränger (50) zur Erzeugung einer peristaltischen Bewegung des Schlauches (18) oder Schlauchabschnitts (44) durch die Durchgangsausnehmung (32) hindurch oder im Bereich der Durchgangsausnehmung (32) in Wirkeingriff mit dem Schlauch (18) oder Schlauchabschnitt (44) bringbar sind.
